(19) Europäisches Patentamt
European Patent Office
Office européen des brevets

(11) Numéro de publication : **0 504 069 A2**

# (12) DEMANDE DE BREVET EUROPEEN

(21) Numéro de dépôt : **92400680.2**

(22) Date de dépôt : **13.03.92**

(51) Int. Cl.[5] : **C07C 57/03,** C07C 51/09, C07C 69/533, C07C 67/333

(30) Priorité : **15.03.91 FR 9103219**

(43) Date de publication de la demande : **16.09.92 Bulletin 92/38**

(84) Etats contractants désignés : **AT BE CH DE DK ES FR GB GR IT LI LU MC NL PT SE**

(71) Demandeur : **ELF SANOFI**
**32-34, rue Marbeuf**
**F-75008 Paris (FR)**

(72) Inventeur : **Bousquet,André**
**Avenue du Lac**
**F-04200 Sistéron (FR)**
Inventeur : **Heymes, Alain**
**Rue Frédéric Mistral 5**
**F-04200 Sistéron (FR)**

(74) Mandataire : **Le Guen, Gérard et al**
**CABINET LAVOIX 2, place d'Estienne d'Orves**
**F-75441 Paris Cédex 09 (FR)**

## (54) Procédé de préparation de l'acide (E)-propyl-2 pentène -2 oique et composés intermédiaires.

(57) L'invention a pour objet une composition oonstituée d'un mélange d'isomères (E) et (Z) d'esters de formule générale :

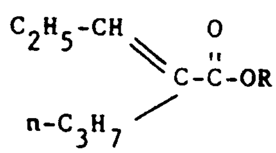

I

dans laquelle R représente un radical alkyle en $C_1$-$C_4$, caractérisé en ce qu'il comporte au moins 85 % d'isomère (E).

L'invention a également pour objet un procédé de préparation d'une telle composition ainsi que son utilisation pour la préparation de l'acide (E)-propyl-2 pentèle-2 oïque de formule :

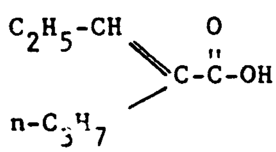

(isomère E) Ia

ainsi que de ses sels pharmaceutiquement acceptables.

EP 0 504 069 A2

La présente invention se rapporte, d'une manière générale, à de nouvelles compositions constituées de mélanges d'isomères d'esters éthyléniques, à leur procédé de préparation ainsi qu'à leur utilisation comme intermédiaires de synthèse.

En particulier, l'invention concerne des compositions constituées de mélanges d'isomères (E) et (Z) d'esters éthyléniques de formule générale :

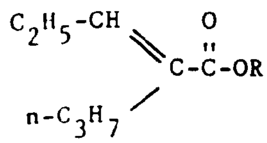

I

dans laquelle R représente un radical alkyle en $C_1$-$C_4$, de préférence le radical éthyle, ces mélanges comportant au moins 85% d'isomère (E).

Les compositions constituées de mélanges d'isomères (E) et (Z) d'esters de formule I comportant au moins 90% d'isomère (E), constituent des compositions préférés de l'invention en particulier la composition constituée d'un mélange comportant 90% d'isomère (E).

Les compositions constituées de mélanges d'isomères (E) et (Z) d'esters de formule I se sont révélées particulièrement utiles comme produits intermédiaires notamment pour la préparation de l'acide (E)-propyl-2 pentène-2 oïque de formule :

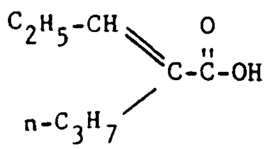

(isomère E) Ia

ainsi que de ses sels pharmaceutiquement acceptables, en particulier le sel sodique.

Cet acide de formule Ia est un produit connu ayant été décrit notamment dans Arch. Pharm. 310 (5) pp. 394-403 (1977) et dans J.A.C.S. 93, (17) pp. 4242-4247 (1971).

Il s'agit d'un métabolite de l'acide valproïque, composé connu pour ses propriétés anticonvulsivantes et largement utilisé dans le traitement de l'épilepsie.

L'acide de formule Ia présente également une activité antiépileptique non négligeable et un degré de tératogénicité inférieur à celui de l'acide valproïque.

On connait déjà plusieurs procédés pour préparer l'acide (E)-propyl-2 pentène-2 oïque.

Cependant, la plupart d'entre eux présentent des inconvénients tels que leur utilisation ne peut être envisagée valablement pour une production sur le plan industriel. En effet, ces différents procédés font intervenir :

– soit des réactifs dangereux tel que le cyanure de sodium, nécessitant une mise en oeuvre délicate,
– soit des composés de départ déjà élaborés et difficilement accessibles tel que l'hydroxy-4 octanone-5,
– soit un nombre d'étapes important avec, en finalité un rendement global faible, de l'ordre de 30%, doublé d'une mise en oeuvre assez lourde en raison des réactifs utilisés.

Il existe cependant des procédés de préparation de l'acide (E)-propyl-2 pentène-2 oïque en question comportant un nombre relativement restreint d'étapes et utilisant des réactifs aisément accessibles. Toutefois, ces procédés présentent des désavantages dont les plus importants se caractérisent par un manque de sélectivité au niveau des isomères de l'acide propyl-2 pentène-2 oïque formé (isomères géométriques et de position) et une séparation difficile de ces isomères entraînant des rendements globaux assez faibles.

A cet égard, on peut citer le procédé décrit dans Arch. Pharm. 310 (1977) cité précédemment, procédé selon lequel :

a) on provoque une déshydrohalogénation du bromo-2 propyl-2 pentanoate d'éthyle, au reflux, au moyen de N,N-diéthylaniline et une purification par distillation (rendement : 76,5%),

b) on saponifie, au reflux, l'ester ainsi obtenu, au moyen d'une solution aqueuse 1,5 M en hydroxyde de sodium et en présence d'éthanol, on traite par l'acide sulfurique et on purifie par cristallisation fractionnée dans l'éther de pétrole (rendement : 25%). Le rendement global de ce procédé s'avère très faible puisque de l'ordre de 19% à partir du bromo-2 propyl-2 pentanoate d'éthyle.

Une modification de cette méthode a été décrite dans la demande de brevet EP-293.753 selon laquelle on provoque :

a) la déshydrohalogénation du bromo-2 propyl-2 pentanoate d'éthyle au moyen de diaza-1,4 bicyclo--

[2,2,2]octane dans l'acétonitrile, la réaction ayant lieu durant 8h à la température de reflux du milieu, pour obtenir après concentration un mélange 77/23 d'isomères (E) et (Z) de propyl-2 pentène-2 oate d'éthyle [rendement : 80% équivalant à un rendement de 62% en isomère (E)],

b) la saponification du mélange d'isomères en question au moyen de 10 équivalents d'hydroxyde de sodium dans le méthanol et durant 5 jours à température ambiante puis l'acidification du milieu pour donner un mélange brut quantitatif 80/20 d'acide (E)-propyl-2 pentène-2 oïque/propyl-2 pentène-3 oïque (isomère Δ 3) et enfin la purification par distillation (rendement non précisé).

En conséquence, le procédé ci-dessus ne permet d'obtenir l'acide (E)-propyl-2 pentène-2 oïque brut qu'avec un rendement maximum de 48%.

En outre, lors de la séparation des isomères formés au niveau de l'acide propyl-2 pentène-2 oïque, on peut s'attendre à d'importants problèmes puisqu'il est précisé dans la demande de brevet en question que la distillation à réaliser pour la purification est délicate et doit être effectuée au moyen d'une colonne puissante.

La mise au point d'un procédé apte à être utilisé sur le plan industriel et permettant de préparer l'acide de formule Ia sous forme pure, c'est-à-dire exempt d'isomères et avec de bons rendements, reste donc d'un intérêt majeur.

Or, on a découvert, de manière surprenante selon l'invention, que les compositions constituées de mélanges d'isomères (E) et (Z) d'esters de formule I peuvent donner accès à l'acide (E)-propyl-2 pentène-2 oïque et à ses sels pharmaceutiquement acceptables avec des rendements de loin supérieurs à ceux fournis par les procédés antérieurs et avec un degré de pureté particulièrement remarquable.

Ces compositions se sont révélées d'autant plus intéressantes qu'elles peuvent être préparées avec facilité et rendements chimiques importants.

En effet, on a trouvé que les compositions de l'invention peuvent être obtenues notamment par simple chauffage d'un ester bromo-2 propyl-2 pentanoïque dans un solvant aprotique polaire comportant un groupement amide tel que le N,N,diméthylformamide, solvant qui se révèle capable, en conséquence, d'induire une réaction de déhydrobromuration (élimination de H Br) au niveau de cet ester contrairement à l'acétonitrile.

En conséquence, l'invention se rapporte à des compositions constituées de mélanges d'isomères (E) et (Z) d'esters de formule I, en tant que produits industriels nouveaux, utiles notamment comme intemédiaires de synthèse par exemple pour la préparation de l'acide (E)-propyl-2 pentène-2 oïque et de ses sels pharmaceutiquement acceptables.

Selon l'invention, on prépare les compositions en question selon un procédé impliquant les étapes suivantes :

a) on traite, dans un solvant polaire, l'acide n-propyl-2 pentanoïque ou acide valproïque, de formule :

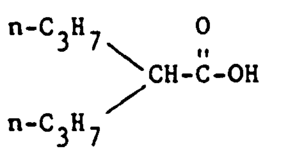

II

d'abord avec le chlorure de thionyle ensuite avec le brome et finalement avec un alcool de formule générale :

R-OH (III)

dans laquelle R a la même signification que précédemment, ces réactions ayant lieu dans un solvant aprotique tel que le dichloréthane, pour obtenir un ester α-bromé de formule générale :

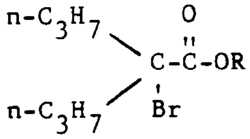

IV

dans laquelle R a la même signification que précédemment,

b) on chauffe l'ester ainsi obtenu à une température de 30°C à 130°C dans un solvant aprotique polaire comportant un groupement amide et éventuellement en présence d'une amine tertiaire, ce qui fournit une composition constituée d'un mélange d'isomères (E) et (Z) de formule I, comportant au moins 85% d'isomère (E).

Préférentiellement, on utilise un alcool de formule III dans lequel R représente le groupement éthyle, c'est-à-dire l'éthanol et un ester α-bromé de formule IV dans lequel R représente également le groupement éthyle

à savoir le bromo-2 propyl-2 pentanoate d'éthyle.

Comme solvant aprotique polaire comportant un groupement amide, on utilise généralement le N,N-diméthylformamide, le N,N-diméthylacétamide ou la N-méthyl pyrrolidone.

Le N,N-diméthylformamide constitue toutefois un solvant préféré.

De plus, le chauffage de l'ester de formule IV dans le solvant comme seul agent de déhydrobromuration s'effectue généralement à une température de 90°C à 130°C préférentiellement à une température de l'ordre de 100°C à 125°C.

Suivant une variante du procédé ci-dessus pour la préparation des compositions de l'invention, on effectue le chauffage de l'ester de formule IV dans le solvant en question en présence d'une amine tertiaire généralement une trialkylamine en $C_1$-$C_4$ telle que la triméthylamine (TMA), la triéthylamine (TEA) ou la triméthyléthylènediamine (TMEDA) ou encore en présence de diaza-1,4 bicyclo[2,2,2]octane (DABCO).

Le solvant préféré, dans cette variante, est également le N,N-diméthylformamide.

Dans ces conditions, le traitement en question s'effectue en présence d'un maximum de 2 moles d'amine tertiaire par mole d'ester de formule IV et généralement à une température de 30°C à 100°C par exemple à une température de 40°C à 70°C mais de préférence à une température de l'ordre de 55°C.

Comme indiqué précédemment, les solvants aprotiques polaires comportant un groupement amide et notamment le N,N-diméthylformamide peuvent agir comme agents de déhydrobromuration, contrairement à l'acétonitrile.

En effet, des essais comparatifs ont été effectués, selon les conditions opératoires de l'invention, en chauffant à 75°C durant 23 heures, l équivalent en poids de bromo-2 propyl-2 pentanoate d'éthyle dans 2 équivalents en volume de solvant à savoir l'acétonitrile, le N,N-diméthylformamide (DMF) ou le N,N-diméthylacétamide (DMAC).

Les résultats ci-dessous rapportent le pourcentage de bromo-2 propyl-2 pentanoate d'éthyle converti en un mélange d'isomères (E) et (Z) de propyl-2 pentène-2 oate d'éthyle, le pourcentage de chacun des isomères obtenus ainsi que le rapport de ces isomères au sein du mélange qu'ils forment.

| Solvant | % de conversion | Isomères | | |
|---|---|---|---|---|
| | | E (%) | Z (%) | E/Z |
| Acétonitrile | 0 | 0 | 0 | 0 |
| DMF | 24,2 | 20,5 | 2,5 | 89/11 |
| DMAC | 38 | 33,3 | 4,4 | 88,3/11,7 |

Ces résultats montrent que, toutes conditions opératoires étant identiques, le DMF et le DMAC induisent une réaction de déhydrobromuration du propyl-2 pentanoate d'éthyle, au contraire de l'acétonitrile, de manière à former un mélange d'isomères (E) et (Z) de propyl-2 pentène-2 oate d'éthyle comportant au moins 85% d'isomère (E).

Les compositions selon l'invention obtenues selon le procédé de l'invention se sont révélées exemptes d'ester propyl-2 pentène-3 oïques correspondant.

En outre, leur rendement au départ de l'ester $\alpha$-bromé de formule IV s'est révélé excellent.

A titre d'exemple, des compositions selon l'invention ont pu être obtenues avec des rendements chimiques généralement supérieurs à 90% à partir du bromo-2 propyl-2 pentanoate d'éthyle et parfois supérieurs à 95%.

Comme indiqué précédemment, les compositions selon l'invention peuvent être valablement utilisées pour la préparation de l'acide (E)-propyl-2 pentène-2 oïque de formule Ia.

En conséquence, un autre objet de l'invention se rapporte à la préparation de l'acide de formule Ia par mise en oeuvre d'un procédé selon lequel on saponifie une composition, selon l'invention, constituée d'un mélange d'isomères (E) et (Z) d'esters de formule I comportant au moins 85% d'isomère (E) et ce, au moyen d'un hydroxyde de métal alcalin dans un milieu réactionnel aqueux contenant un alcool de formule III, puis en acidifiant le milieu au moyen d'un acide fort, pour obtenir l'acide (E)-propyl-2 pentène-2 oïque.

L'acide obtenu selon cette méthode possède un degré de pureté important puisque son titre en isomère (E) pur est généralement de l'ordre de 95 à 96%.

Préférentiellement, on utilise une composition constituée d'un mélange d'isomères (E) et (Z) de propyl-2

pentanoate d'éthyle et l'éthanol comme alcool de formule III.

La saponification a généralement lieu en présence de 1 à 10 équivalents, de préférence de 1 à 5 équivalents, d'un hydroxyde de métal alcalin, de préférence l'hydroxyde de sodium.

Cette réaction de saponification se déroule en général à une température allant de la température ambiante à 100°C, par exemple à une température de 30°C à 100°C.

En outre cette réaction de saponification s'effectue dans un milieu réactionnel formé d'un milieu aqueux contenant un alcool de formule III, préférentiellement de 50 à 150% en volume par exemple 100% par volume d'eau ainsi que de préférence de 2 à 10 moles/l d'hydroxyde de métal alcalin, par exemple de 3 à 8 moles/l et généralement de 1 à 5 moles d'une composition de l'inventiontel que 1 à 3 moles/l de préférence 1,5 mole/l.

Quant à l'acidification, celle-ci est généralement menée à une température de +20°C à -10°C, de préférence à une température de 0°C à -10°C.

Selon cette méthode, l'acide (E)-propyl-2 pentène-2 oïque peut être obtenu avec excellent rendement chimique puisque de l'ordre de 95% à partir de la composition de l'invention. En outre, le rendement global s'avère de loin supérieur à celui obtenu selon les techniques antérieures puisqu'il est d'environ 90% à partir de l'isomère (E) de formule I et de l'ordre de 75% à partir du dérivé α-bromo de formule IV.

Au surplus, le degré de pureté de l'acide de formule Ia ainsi obtenu s'avère très important puisque supérieur à 95%.

Si nécessaire, la purification de cet acide de formule Ia peut être améliorée par une ou plusieurs recristallisations dans un milieu aqueux contenant un alcool de formule III, milieu analogue à celui défini précédemment pour la saponification.

Généralement une seule recristallisation suffit pour obtenir un produit de pureté très élevée puisque titrant au moins 98% en isomère (E) [isomère (Z) $< 1,5\%$ et isomère $\Delta_3 < 0,3\%$].

Après purification, le rendement en isomère (E) de l'acide propyl-2 pentène-2 oïque est d'environ 72% à partir de l'ester α-bromé de formule IV.

La préparation de l'acide de formule Ia, par saponification au départ d'une composition constituée d'un mélange d'isomères (E) et (Z) d'esters de formule I selon l'invention, peut être réalisée selon une variante de la méthode décrite précédemment.

Cette variante repose notamment sur la mise en évidence d'une cinétique de saponification des isomères (E) et (Z) des esters propyl-2 pentène-2 oïques de formule I totalement différente puisqu'il a été constaté que l'isomère (E) se saponifie beaucoup plus rapidement que l'isomère (Z) correspondant.

En conséquence, cette différence peut être mise à profit en contrôlant efficacement le taux de saponification de manière à limiter la conversion à un mélange maximal en sel d'acide (E)-propyl-2 pentène-2 oïque et minimal en sel d'acide (Z)-propyl-2 pentène-2 oïque.

Cette variante de la méthode de saponification selon l'invention, s'est révélée particulièrement efficace lors de sa mise en oeuvre à partir d'une composition constituée d'un mélange d'isomères (E) et (Z) d'esters de formule I, selon l'invention.

Selon la variante en question, on prépare l'acide (E)-propyl-2 pentène-2 oïque :

a) en saponifiant jusqu'à un taux de conversion de 90 à 92%, une composition, selon l'invention, constituée d'un mélange d'isomères (E) et (Z) d'esters éthyléniques de formule I, la saponification étant effectuée au moyen d'un hydroxyde de métal alcalin dans un milieu réactionnel aqueux contenant un alcool de formule III, en extrayant les esters non saponifiés au moyen d'un solvant non miscible à l'eau par exemple un éther, et en acidifiant le milieu réactionnel aqueux après extraction des esters non saponifiés, au moyen d'un acide fort, pour obtenir essentiellement l'acide (E)-propyl-2 pentène-2 oïque

b) en purifiant l'acide pentène-2 oïque obtenu, par une ou plusieurs cristallisations dans l'eau et en présence d'un alcool de formule III, ce qui fournit l'acide (E)-propyl-2 pentène-2 oïque sous forme particulièrement pure.

La saponification a généralement lieu en présence de 2 à 10 équivalents, de préférence 5 équivalents d'un hydroxyde de métal alcalin. Préférentiellement, on utilise comme hydroxyde de métal alcalin, l'hydroxyde de sodium.

Cette réaction de saponification se déroule en général à une température de 30 à 50°C, de préférence à une température de l'ordre de 40°C dans un milieu réactionnel formé d'un milieu aqueux contenant un alcool de formule III, préférentiellement de 50 à 150% en volume, par exemple 100%, par volume d'eau ainsi que, de préférence, de 2 à 10 moles/l d'hydroxyde de métal alcalin, par exemple 7,5 moles/l et de préférence de 1 à 3 moles/l, d'une composition d'esters de formule I, selon l'invention. Quant à l'acide fort, celui-ci est généralement un hydracide tel que l'acide chlorhydrique.

Le procédé ainsi décrit permet d'obtenir l'acide de formule Ia avec des rendements voisins de 85% à partir de l'ester (E) de formule I et de l'ordre de 75% en produit repurifié calculés à partir de l'ester de formule IV. En outre, le produit pur ainsi obtenu présente un titre en acide (E)-propyl-2 pentène-2 oïque supérieur à 98% voire

supérieur à 98,7%.

Comme indiqué précédemment, la supériorité du procédé de l'invention, sur l'état de la technique, repose notamment sur la mise en évidence d'une cinétique de saponification des isomères (E) et (Z) des esters propyl-2 pentène-2 oïques de formule I.

Cette différence de cinétique est nettement perceptible sur la Figure ci-annexée. Sur cette figure on a représenté, à titre d'exemple :

– les courbes de cinétique de saponification des isomères (E) (courbe a) et (Z) (courbe b) du propyl-2 pentène-2 oate d'éthyle au moyen de 5 équivalents d'hydroxyde de sodium à 40°C et dans un milieu eau/éthanol

– la courbe de cinétique de formation du mélange correspondant des sels sodiques comportant des sels sodiques des isomères (E) et (Z) de l'acide propyl-2 pentène-2 oïque et de l'isomère Δ 3 (courbe c).

Ainsi, en limitant la réaction de saponification à un taux de conversion voisin de 90%, on peut réduire de moitié le pourcentage d'isomère (Z) contenu dans l'acide propyl-2 pentène-2 oïque brut par rapport à l'isomère (Z) contenu dans l'ester propyl-2 pentène-2 oïque de formule I sans accroître le taux d'isomère Δ 3.

Quant aux sels pharmaceutiquement acceptables de l'acide (E)-propyl-2 pentène-2 oïque, ceux-ci peuvent être obtenus par réaction dans un solvant approprié tel que le toluène, avec un agent basique approprié par exemple un hydroxyde de métal alcalin tel que l'hydroxyde de sodium ou un carbonate de métal alcalin tel que le carbonate de sodium, pour former un sel pharmaceutiquement acceptable, de préférence le sel de sodium.

Les Exemples, non limitatifs suivants, illustrent les compositions de l'invention ainsi que leur utilisation :

## EXEMPLE 1

### Préparation d'une composition constituée d'un mélange d'isomères (E) et (Z) de propyl-2 pentène-2 oate d'éthyle.

a) Préparation du bromo-2 propyl-2 pentanoate d'éthyle

Dans un réacteur de Grignard, on introduit, sous atmosphère d'azote, 144g d'acide propyl-2 pentanoïque et 0,5g de N,N-diméthylformamide.

On chauffe l'ensemble à 65°C et on additionne, en 8h, 128,4g de chlorure de thionyle en maintenant la température du milieu entre 65 et 70°C. En fin d'addition, on chauffe le milieu à 95°C et on maintient cette température durant une heure. On introduit ensuite 40g de dichloro-1,2 éthane, chauffe à 100°C et additionne en 8h, 164g de brome. On maintient la température du milieu à 110-115°C durant une heure après la fin de l'addition, on refroidit le mélange réactionnel à 80-85°C, puis on ajoute, en 2h 30 min., 74g d'éthanol absolu. On chauffe à reflux durant une heure, puis on refroidit à 20°C avant d'additionner 40g d'eau. On décante, on concentre la phase organique et distille le résidu huileux [P.Eb : 117-119°C (20 mmHg)].

De cette manière, on obtient 242g de bromo-2 propyl-2 pentanoate d'éthyle. Rendement : 96,5%.

b) Préparation d'un mélange d'isomères (E) et (Z) du propyl-2 pentène-2 oate d'éthyle

Dans un réacteur de Grignard de 500ml, on introduit 0,43 mole d'amine, 320ml de N,N-diméthylformamide et 53g (0,21 mole) de bromo-2 propyl-2 pentanoate d'éthyle. On chauffe le milieu réactionnel à une température T durant X heures. On refroidit alors le mélange à 20°C et on ajoute 100ml d'eau puis 50ml d'acide chlorhydrique concentré. On laisse le milieu sous agitation durant 1 heure puis on extrait par 3 fois 400ml d'hexane. On lave les phases organiques par 3 fois 350ml d'eau, sèche sur sulfate de sodium et concentre.

De cette manière, on obtient un mélange d'isomères (E) et (Z) de propyl-2 pentène-2 oate d'éthyle sous forme d'un résidu huileux incolore exempt de propyl-2 pentène-3 oate d'éthyle.

En utilisant le procédé décrit ci-dessus, on a obtenu les résultats suivants :

| Amine | T (°C) | X (h) | Rendement brut (%) | Rapport des isomères E/Z* |
|---|---|---|---|---|
| DABCO | 55 | 21 | 96,7 (35 g) | 89/11 |
| DABCO | 90 | 4,5 | 97 | 86/14 |

En utilisant le même procédé que celui décrit précédemment mais en chauffant le milieu réactionnel à une température $T_1$ durant $X_1$ heures puis à une température $T_2$ durant $X_2$ heures, on a obtenu les résultats suivants :

| Amine | $T_1$(°C)<br>$T_2$(°C) | $X_1$(h)<br>$X_2$(h) | Rendement brut (%) | Rapport des isomères E/Z* |
|---|---|---|---|---|
| TMA | 55<br>80 | 44<br>4 | 89 | 89/11 |
| TEA | 55<br>100 | 16<br>22 | 91 | 88/12 |
| TMEDA | 55<br>100 | 17<br>9 | 90 | 85/15 |

* déterminé par résonance magnétique nucléaire (R.M.N.) dans $CDCl_3$ (300 MHz) : triplet à 6,75 et 5,80 ppm.

**EXEMPLE 2**

**Préparation d'une composition constituée d'un mélange d'isomères (E) et (Z) de propyl-2 pentène-2 oate d'éthyle.**

En suivant le même processus opératoire que celui décrit à l'Exemple 1, on convertit en totalité 100g de bromo-2 propyl-2 pentanoate d'éthyle dans 200ml de N,N-diméthylformamide sans ajout d'amine tertiaire et ce, à 125°C durant 15 heures.

De cette manière, on obtient un mélange d'isomères (E) et (Z) de propyl-2 pentène-2 oate d'éthyle dans un rapport E/Z : 85/15.

**EXEMPLE 3**

**Préparation de l'acide (E)-propyl-2 pentène-2 oïque**

a) Saponification

Dans un réacteur de Grignard, on introduit, sous argon, 20,4 ml d'eau, 12g d'hydroxyde de sodium en pastilles et 20,4ml d'éthanol. On chauffe l'ensemble à 40°C et on additionne rapidement 10,21g (0,06 mole) d'un mélange 90/10 d'isomères (E)/(Z) de propyl-2 pentène-2 oate d'éthyle. On maintient le milieu réactionnel à cette température pendant 130 minutes. La conversion d'ester en acide est alors d'environ 90% (contrôle par chromatographie en phase gazeuse). On additionne au milieu réactionnel 100ml d'eau épurée et 30ml d'éther diisopropylique. On décante et extrait une nouvelle fois la phase aqueuse par 30ml d'éther diisopropylique. On acidifie la phase aqueuse par 35ml d'acide chlorhydrique à 37% et extrait par 3 fois 50ml d'éther diisopropylique. On rassemble les phases organiques et les lave par 3 fois 50ml d'eau. On sèche sur sulfate de sodium et concentre à l'évaporateur rotatif (température $\leqq$ 30°C).

De cette manière, on obtient 7,7g d'acide (E)-propyl-2 pentène-2 oïque. Rendement : 90,3%.

Par R.M.N. dans $CDCl_3$ (300 MHz), on détermine le rapport isomérique E/Z = 94/6 (triplet à 6,8 et 6 ppm).

b) Isolement de l'acide (E)-propyl-2 pentène-2 oïque

Dans un ballon tricol de 100 ml, on introduit 22ml d'éthanol, 33ml d'eau et 7,6g d'acide (E)-propyl-2 pentène-2 oïque obtenu précédemment. On chauffe au reflux puis refroidit le milieu, lentement en 1 heure, de 30°C à -10°C. L'acide (E)-propyl-2 pentène-2 oïque précipite vers 0°C. Après filtration, on recristallise, dans les mêmes conditions le précipité obtenu puis on réempâte par un mélange eau/éthanol 70/30.

De cette manière, on obtient 6,5g d'acide (E)-propyl-2 pentène-2 oïque.

Rendement par rapport au (E)-propyl-2 pentène-2 oate d'éthyle : 85,5% Rendement par rapport au bromo-2 pentanoate d'éthyle : 74,2% Pureté déterminée par chromatographie en phase gazeuse capillaire ( CPG) :

titre 98,9% en isomère (E)
P.F. : 38°C
Impuretés :
Isomère (Z) : 0,7%
Isomère $\Delta_3$ : 0,4%

**EXEMPLE 4**

**Préparation de l'acide (E)-propyl-2 pentène-2 oïque**

a) Saponification

Dans un réacteur de Grignard, on charge à la température de 20°C, 1,0 Kg (5,88 moles) d'un mélange 87/13 d'isomères (E)/(Z) de propyl-2 pentène-2 oate d'éthyle (P.E. 75°C sous 10mm Hg; d = 0,92 à 20°C; rendement : 97% par rapport au bromo-2 propyl-2 pentanoate d'éthyle) 0,48 Kg (607ml) d'éthanol, 0,6 Kg (600ml) d'eau et 0,271 Kg (6,775 moles) d'hydroxyde de sodium en écailles. On porte le mélange jusqu'à reflux franc durant 1 heure et on l'y maintient durant 1 heure. On refroidit à 20°C puis on coule en 1 heure 0,817 Kg (8,06 moles) d'acide chlorhydrique à 36% tout en poursuivant le refroidissement vers 0°C. On maintient le mélange sous agitation à 0°C pendant 1 heure puis on refroidit à -10°C pendant 1 heure. On essore, rince le précipité

d'abord au moyen d'un mélange, glacé à -10°C, formé de 0,33 Kg d'éthanol et 0,84 Kg d'eau, ensuite au moyen de 1,2 Kg d'eau glacée à 0° à 5°C.

On sèche alors à 20°C sous vide ou en étuve ventilée.

De cette manière, on obtient 0,650 Kg d'acide (E)-propyl-2 pentène-2 oïque sous forme d'un liquide limpide incolore.

Rendement : 89,6% par rapport au (E)-propyl-2 pentène-2 oate d'éthyle ou 75,6% par rapport au bromo-2 propyl-2 pentanoate d'éthyle.

Pureté déterminée par C.P.G. : titre 96% en isomère (E)

Impuretés :

Isomère (Z) : 2,8%

Isomère $\Delta_3$ : 1,1%

b) Isolement de l'acide (E)-propyl-2 pentène-2 oïque

Dans un réacteur de Grignard, on dissout, à 70°C, 0,650 Kg d'acide (E)-propyl-2 pentène-2 oïque, obtenu précédemment, dans un mélange de 0,75 Kg (950ml) d'éthanol et 0,95 Kg (950ml) d'eau. On refroidit à 0°C et on maintient durant 1 heure à cette température. On refroidit à -10°C pendant 1 heure, on essore et on rince au moyen d'un mélange formé de 0,155 Kg (196ml) d'éthanol et 0,2 Kg (200ml) d'eau épurée, mélange préalablement glacé à -10°C.

On sèche alors à 20°C sous vide ou en étuve ventilée.

De cette manière, on recueille 0,580 Kg d'acide (E)-propyl-2 pentène-2 oïque sous forme d'une poudre blanche cristalline. Rendement de purification : 89%, ce qui correspond à un rendement de 80% par rapport au (E)-propyl-2 pentène-2 oate d'éthyle ou un rendement de 67% par rapport au bromo-2 propyl-2 pentanoate d'éthyle.

Pureté déterminée par CPG : titre 99,6% en isomère (E)

Impuretés : Isomère (Z) : 0,2%

0,2% (artefact)

## EXEMPLE 5

### Préparation de l'acide (E)-propyl-2 pentène-2 oïque

a) Saponification

En utilisant le même procédé qu'à l'Exemple 4a au départ de 1,0 Kg (5,88 moles) d'un mélange 88/12 d'isomères (E)/(Z) de propyl-2 pentène-2 oate d'éthyle (rendement : 96% par rapport au bromo-2 propyl-2 pentanoate d'éthyle), on obtient 0,660 Kg d'acide (E)-propyl-2 pentène-2 oïque. Rendement : 90% par rapport au (E)-propyl-2 pentène-2 oate d'éthyle ou 76% par rapport au bromo-2 propyl-2 pentanoate d'éthyle.

b) Isolement de l'acide (E)-propyl-2 pentène-2 oïque

En utilisant le même procédé qu'à l'Exemple 4b, on isole 0,620 Kg d'acide (E)-propyl-2 pentène-2 oïque Rendement de purification : 94%, ce qui correspond à un rendement de 84,4% par rapport au (E)-propyl-2 pentène-2 oate d'éthyle ou un rendement de 71,5% par rapport au bromo-2 propyl-2 pentanoate d'éthyle.

Pureté déterminée par CPG : titre 99,8% en isomère (E)

Impuretés : Isomère (Z) : 0,01%

0,01% (artefact)

## Revendications

1. Composition constituée d'un mélange disomères (E) et (Z) d'esters de formule générale :

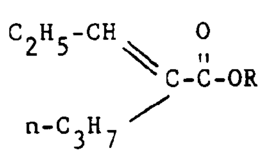

I

dans laquelle R représente un radical alkyle en $C_1$-$C_4$, caractérisé en ce qu'il comporte au moins 85% d'isomère (E).

2. Composition selon la Revendication 1 comportant 90% d'isomère (E).

3. Composition selon la Revendication 1 ou 2 caractérisé en ce que R représente le radical éthyle.

4. Procédé de préparation d'une composition selon la Revendication 1, caractérisé en ce que :
a) on chauffe un ester α-bromé de formule générale :

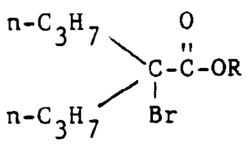

IV

dans laquelle R a la même signification que dans la Revendication 1 à une température de 30°C à 130°C dans un solvant aprotique polaire comportant un groupement amide et éventuellement en présence d'une amine tertiaire, ce qui fournit la composition désirée.

5. Procédé de préparation de l'acide (E)-propyl-2 pentène-2 oïque de formule :

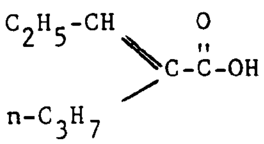

(isomère E) Ia

ainsi que de ses sels pharmaceutiquement acceptables, caractérisé en ce que l'on saponifie une composition selon la Revendication 1 au moyen d'un hydroxyde de métal alcalin dans un milieu réactionnel aqueux contenant un alcool de formule générale :

R-OH (III)

dans laquelle R a la même signification que précédemment, puis en acidifiant le milieu au moyen d'un acide fort pour obtenir essentiellement l'acide (E)-propyl-2 pentène-2 oïque désiré.

6. Procédé de préparation de l'acide (E)-propyl-2 pentène-2 oïque de formule :

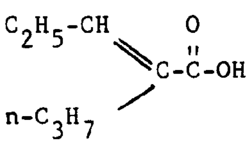

(isomère E) I a

ainsi que de ses sels pharmaceutiquement acceptables, caractérisé en ce que :
l'on saponifie, jusqu'à un taux de conversion de 90 à 92%, un mélange d'isomère (E) et (Z), comportant au moins 85% d'isomère (E), d'esters de formule générale :

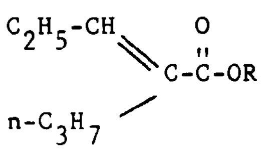

II

dans laquelle R représente un radical alkyle en $C_1$-$C_4$, au moyen d'un hydroxyde de métal alcalin dans un milieu réactionnel contenant un alcool de formule générale :

R-OH (III)

dans laquelle R a la même signification que précédemment, on extrait les esters non saponifiés au moyen d'un solvant non miscible à l'eau et on acidifie le milieu réactionnel aqueux après extraction des esters non saponifiés, au moyen d'un acide fort, pour obtenir essentiellement l'acide (E)-propyl-2 pentène-2 oïque désiré.

**7.** Procédé de préparation de l'acide (E)-propyl-2 pentène-2 oïque de formule :

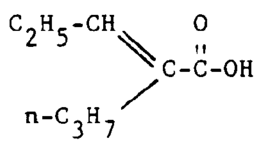

(isomère E) Ia

ainsi que de ses sels pharmaceutiquement acceptables, caractérisé en ce que :
a) on chauffe un ester α-bromé de formule générale :

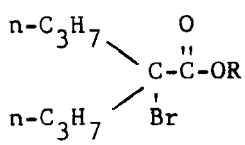

IV

dans laquelle R a la même signification que dans la Revendication 1, à une température de 30°C à 130°C dans un solvant aprotique polaire comportant un groupement amide et éventuellement en présence d'une amine tertiaire, ce qui fournit une composition constituée d'un mélange d'isomères (E) et (Z) d'esters de formule générale :

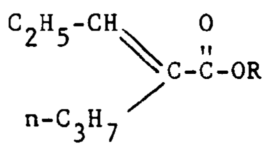

I

dans laquelle R représente un radical alkyle en $C_1$-$C_4$, mélange comportant au moins 85% d'isomère (E)
b) on saponifie la composition ainsi obtenue au moyen d'un hydroxyde de métal alcalin dans un milieu réactionnel aqueux contenant un alcool de formule générale :

R-OH (III)

dans laquelle R a la même signification que précédemment, puis on acidifie le milieu au moyen d'un acide fort pour obtenir essentiellement l'acide (E)-propyl-2 pentène-2 oïque désiré.

**8.** Procédé selon l'une des Revendications 4 à 6 caractérisé en ce que l'on purifie l'acide (E)-propyl-2 pentène-2 oïque par une ou plusieurs cristallisations dans un milieu aqueux contenant un alcool de formule III, ce qui fournit l'acide (E)-propyl-2 pentène-2 oïque sous forme pure.

**9.** Procédé selon la Revendication 8 caractérisé en ce que l'on effectue la purification dans un milieu aqueux contenant de 50 à 150% en volume d'un alcool de formule III.

**10.** Procédé selon la Revendication 4 ou 7 caractérisé en ce que l'on chauffe l'ester α-bromé de formule IV, à une température de 100 à 125°C , dans un solvant aprotique polaire comportant un groupement amide.

**11.** Procédé selon la Revendication 4 ou 7 caractérisé en ce que l'on chauffe l'ester α-bromé de formule IV, à une température de 30°C à 100°C, dans un solvant aprotique polaire comportant un groupement amide et en présence d'une amine tertiaire.

**12.** Procédé selon une des Revendications 4, 7, 10 ou 11, caractérisé en ce que le solvant aprotique polaire

comportant un groupement amide est le N,N-diméthylformamide, le N,N-diméthylacétamide ou la N-méthyl pyrrolidone.

**13.** Procédé selon une des Revendications 4, 7 ou 11 caractérisé en ce que l'on utilise un maximum de 2 moles d'amine tertiaire par mole d'ester α-bromé de formule IV.

**14.** Procédé selon une des Revendications 4, 7, 11 ou 13 caractérisé en ce que l'amine tertiaire est une trialkylamine en $C_1$-$C_4$.

**15.** Procédé selon une des Revendications 4, 7, 11, 13 ou 14 caractérisé en ce que l'amine tertiaire est la triméthylamine, la triéthylamine ou la triméthyléthylènediamine.

**16.** Procédé selon une des Revendications 4, 7, 11 ou 13 caractérisé en ce que l'amine tertiaire est le diaza-1,4 bicyclo[2,2,2]octane.

**17.** Procédé selon la Revendication 4 ou 7 caractérisé en ce que l'ester α-bromé de formule IV est le bromo-2 propyl-2 pentanoate d'éthyle.

**18.** Procédé selon une des Revendications 5 à 7 caractérisé en ce que la saponification a lieu à une température allant de la température ambiante à 100°C.

**19.** Procédé selon une des Revendications 5 à 7 caractérisé en ce que l'on effectue la saponification au moyen de 1 à 10 équivalents d'hydroxyde de métal alcalin.

**20.** Procédé selon une des Revendications 5 à 7 caractérisé en ce que l'hydroxyde de métal alcalin est l'hydroxyde de sodium.

**21.** Procédé selon une des Revendications 5 à 7 caractérisé en ce que le milieu réactionnel aqueux contient de 50 à 150% en volume d'un alcool de formule III.

**22.** Procédé selon une des Revendications 5 à 7 caractérisé en ce que l'acidification a lieu à une température de +20°C à -10°C.

**23.** Procédé selon une des Revendications 5 à 7 caractérisé en ce que l'acide fort est l'acide chlorhydrique.

**24.** Procédé selon une des Revendications 5 à 7 caractérisé en ce que l'on fait réagir l'acide (E)-propyl-2 pentène-2 oïque avec un hydroxyde de métal alcalin ou un carbonate de métal alcalin pour obtenir un sel pharmaceutiquement acceptable.

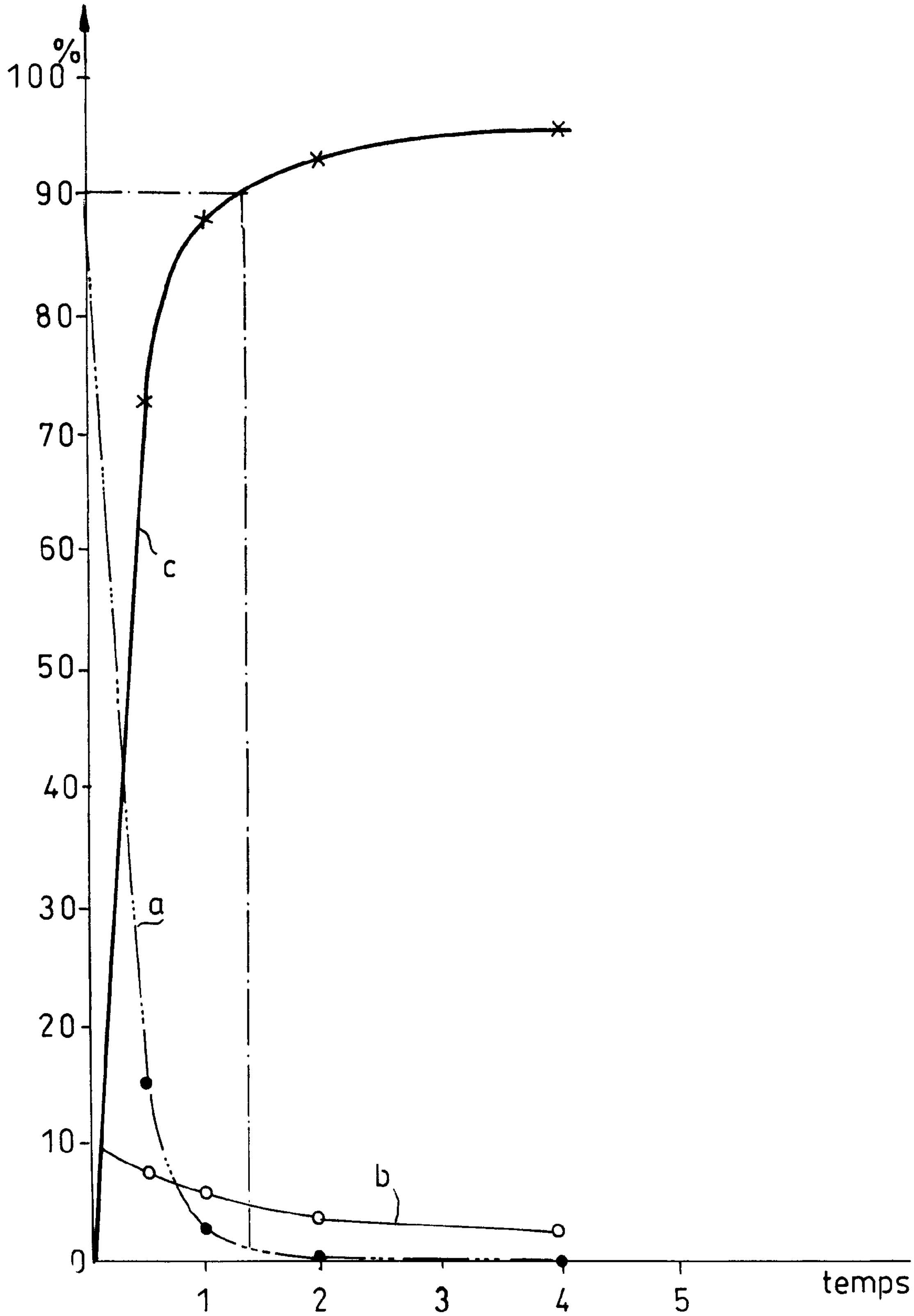
%
100
90
80
70
60
50
40
30
20
10
0
c
a
b
1
2
3
4
5
temps